# EUROPEAN PATENT APPLICATION

(11) **EP 2 228 438 A1**
(43) Date of publication of application: **15.09.2010**
(21) Application number: 09154389.2
(22) Date of filing: 05.03.2009
(51) Int. Cl.: C12N 5/00, C12M 1/00

(54) **Soluble poly(amido-amines) as promoters of cell adhesion, proliferation and differentiation**

(71) Applicant: UNIVERSITA' DEGLI STUDI DI MILANO, 20122 Milano (IT); Neuro- Zone S.r.l., 20122 Milano (IT)
(72) Inventor: Ranucci, Elisabetta, 20090 Opera (MI) (IT); Ferruti, Paolo, 20145 Milano (IT); Lenardi, Cristina, 21020 Casciago (VA) (IT); Matteoli, Michela, 20047 Brugherio (MI) (IT)
(74) Representative: Minoja, Fabrizio

(57) **Abstract**

Use of soluble polyamidoamines having a molecular weight comprised from about 3.000 Da to about 120.000 Da, more preferably from about 5.000 Da to about 40.000 Da, to promote adhesion, growth and differentiation of cultured cell lines and primary cell cultures on cell-growth supporting substrates.

## Description

The present invention relates to polyamidoamines and to their use to promote adhesion, proliferation and differentiation of cell lines or primary cells such as neurons, astrocytes and stem cells from rodent, on plates or other cell-culture substrates.

Linear polyamidoamines are known synthetic tert-amino polymers obtained by stepwise polyaddition of primary or secondary aliphatic amines or aminoacids to bis(acrylamide)s, They consist of a backbone chain having amido- and tertiary amino groups that are arranged regularly along the backbone. Depending on the amine used in the polyaddition step, they may possess purely basic or amphoteric properties. Amphoteric polyamidoamines, in turn, may have isoelectric points ranging from 3 to 10. Polyamidoamines can be synthesised from a wide variety of primary or secondary amines, which enables full control to be exercised over the spacing and pKa of groups along the backbone.

Cationic polymers such as polyamidoamines have potential applications in many areas due to their positive charge. Polyamidoamines are highly hydrophilic, and usually degrade in aqueous media at a rate depending on their structure. The combination of beneficial physicochemical properties and good biocompatibility makes polyamidoamines a suitable choice for their use in various biological and medical applications. For example, polyamidoamines can act as useful polymers for the preparation of polyelectrolyte complexes for the delivery of biomolecule payloads, such as DNA constructs or anticancer drugs, to a target site in a patient.

Other useful applications of polyamidoamines comprise the use of polyamidoamine complexes in the delivery and protection of active payload biomolecules in the environment, their use as heavy-metal ion complexing polymers or for heparin neutralisation in solution, fabrication of heparin-absorbing resins and heparinisable materials.

Due to their hydrophilic nature, when cross-linked, polyamidoamines can form hydrogels which may act as scaffold structures in tissue engineering applications. Compositions comprising a polyamidoamine polymer containing a pendant disulphide, sulphydryl, or activated sulphydryl moiety are disclosed in WO 2008/038038. This application discloses also the use of such polyamidoamine polymers to form cross-linked compositions and hydrogels and the use of such compositions in various biological and non-biological applications, such as the delivery of biomolecules to target sites, and for tracking fluid flows.

Thick films of hydrogels based on a slightly polycationic polyamidoamine have been reported to be able to promote cellular proliferation and adhesion. However, the reticulated and insoluble nature of these cross-linked hydrogels prevents their use as substrates for cell adhesion and proliferation with the usual procedures and equipment employed in cell culture laboratories.

Cationic polymers such as poly-L-ornithine, poly-D-lysine and poly-L-lysine have been used as cell adhesion, proliferation and differentiation substrates for many years, but their toxicity makes them problematic for this purpose. The procedures known in the art to obtain cellular cultures requires the treatment of plastic or glass plates suitable to support cell growth with aqueous solutions of basic polyaminoacids like poly-lysines and poly-ornithines. The polymer-treated plates are then subjected to repeated and prolonged washing cycles to remove the excess of the cationic polymer, which otherwise would be toxic to the cells, and to plate drying under sterile conditions. Only after these lengthy steps, the plates can be used to support cellular growth, adhesion and differentiation in cell cultures. The main problems associated with such procedures are the high costs of the reagents, the narrow windows of useful polyamine concentrations due to their inherent cytotoxic effects and the long times required to obtain a plate ready for the intended use, which may range from one day to one week depending on the cell line.

No examples are known of synthetic polymers that can effectively replace poly-L-Lysine, poly-D-Lysine or poly-L-Ornithine for cell culture applications.

It has now surprisingly been found that known soluble polyamidoamines according to this invention are a convenient alternative to standard basic polyaminoacids for cell culture applications, avoiding all the drawbacks associated with the use of the latter polymers. In particular, soluble polyamidoamines according to the invention have much lower cellular toxicity than the basic polyaminoacids currently used and they allow for simplified and quicker procedures for cell-culture purposes. Finally, they are much less expensive than the basic polyaminoacids.

A first object of this invention is therefore the use of soluble polyamidoamines as promoters for cell adhesion, proliferation or differentiation of cell lines or primary cells such as neurons, astrocytes and stem cells from rodents, on plates or other cell-culture substrates.

The soluble polyamidoamines that can be used for the purposes of the present invention are known compounds. Any known soluble polyamidoamine having a molecular weight comprised from about 3.000 Da to about 120.000 Da, more preferably from about 5.000 Da to about 40.000 Da can be used. It can be reasonably expected that the ability of promoting adhesion and proliferation of some cell lines is a general property of polyamidoamines. Examples of such compounds are described in Macromol. Rapid Commun. 2002, 23, 332-335 and references cited therein. Preferred examples of polyamidoamines that can be used according to the invention are:
1) Basic polyamidoamines obtained by polyaddition of neutral bisacrylamides and amines and having from mild to strong cationic properties at pH 7.4, such us, for example the polyamidoamines having as repeating units the structures of formula (I), formula (II) and formula (III): 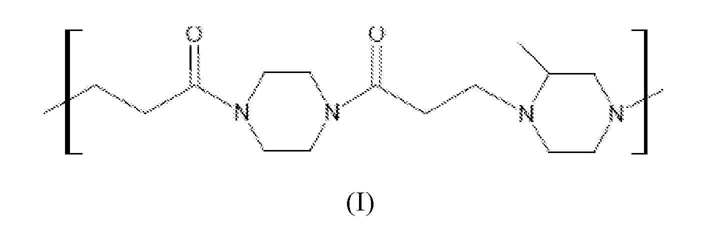 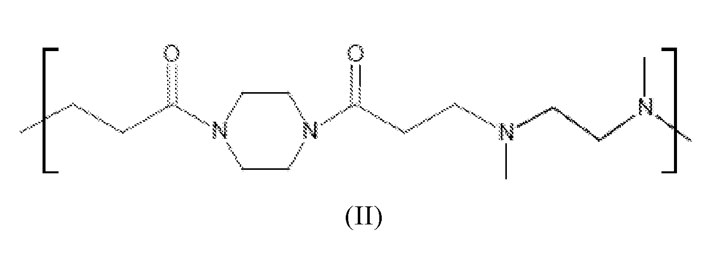 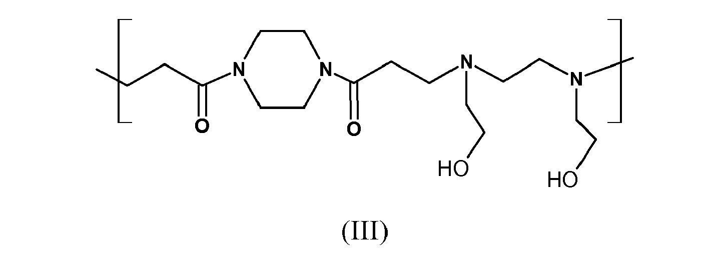
2) Amphoteric polyamidoamines obtained by polyaddition of bisacrylamides substituted with acidic groups and amines that may have a prevailing acidic nature and an isoelectric point lower than 7.0, owing to the weak basicity of the ter-amino groups and the strong acidity of the carboxyl group. Examples of such polyamidoamines are those having as repeating units the structures of formula (IV) and formula (V): 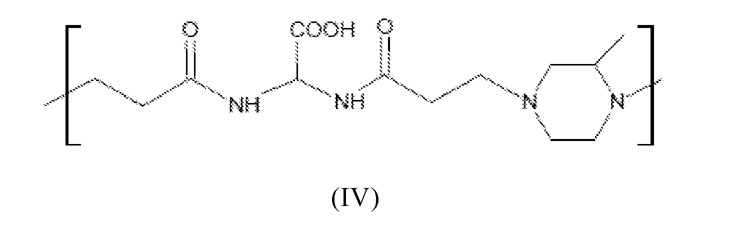 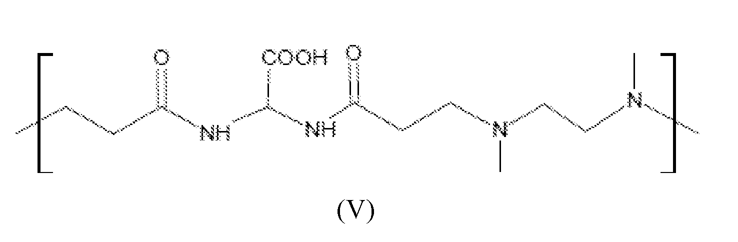
3) Amphoteric polyamidoamines obtained by polyaddition of bisacrylamides substituted with neutral or acidic-groups and aminoacids, which have a prevailing acidic nature and may have an isoelectric point lower than 7.0. Examples of such polyamidoamines are those having as repeating units the structures of formula (VI), (VII), (VIII) and (IX): 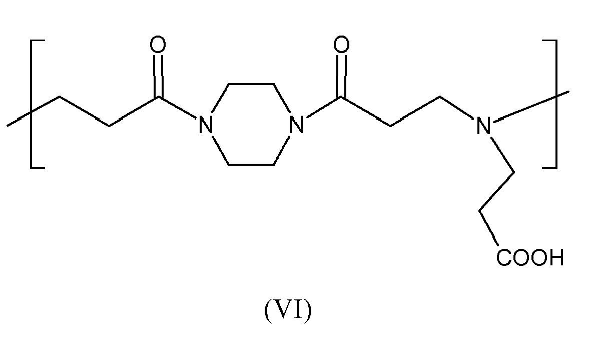 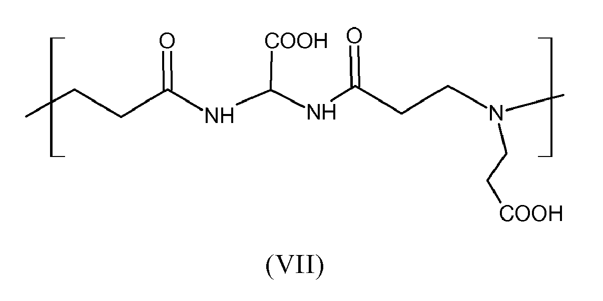 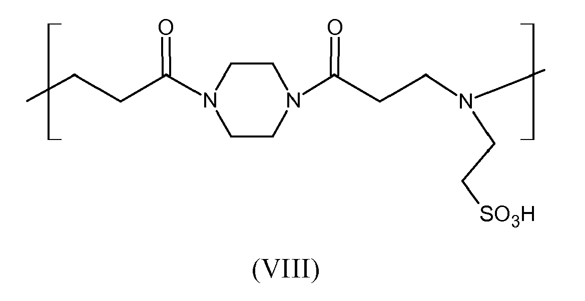 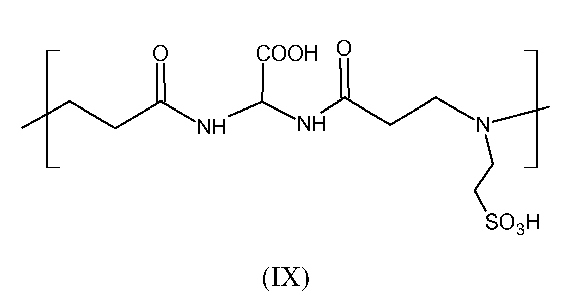
4) Polyamidoamines containing residues of agmatine, obtained by polyaddition of bisacrylamides substituted with neutral or acidic-groups and agmatine, which have a prevailing basic nature and, if amphoteric, may have an isoelectric point higher than 7.0, such as, for example AGMA1 of formula (X): 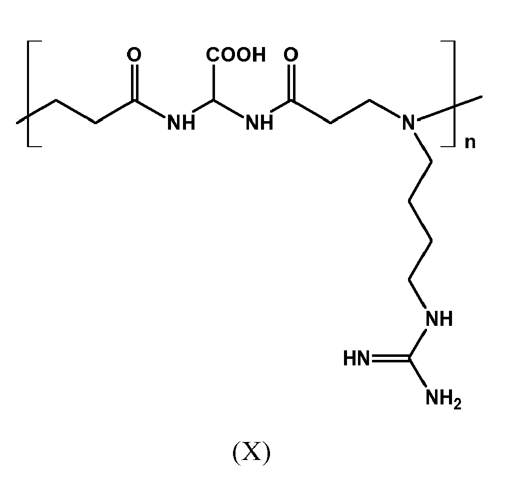 wherein n is an integer such that the molecular weight of the polyamidoamine is comprised from about 3.000 Da to about 120.000 Da, more preferably from about 5.000 Da to about 40.000 Da.
5) Copolymeric polyamidoamines, such as for example ISA1 of formula (XI): 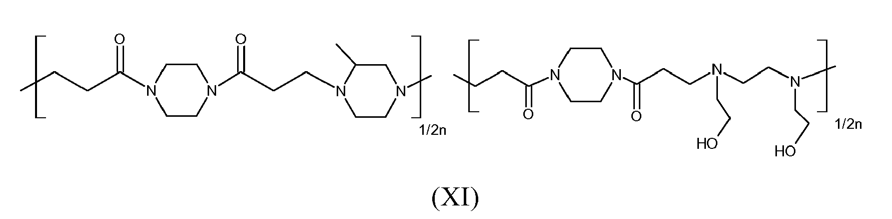 wherein n is an integer such that the molecular weight of the copolymeric polyamidoamine is comprised from about 3.000 Da to about 120.000 Da, more preferably from about 5.000 Da to about 40.000 Da.

Polyamidoamines having as repeating units the structures of formulae (I) - (IX) can be prepared according to P. Ferruti, Polymer 1985, 26, 1336-1348 and Macromolecules 2000, 33, 7793-7800 and according to M. Rossi, "Electroanalysis for Physico-Chemical Characterization and Molecular Design of Polymers of Biomedical Interest: Poly(amidoamine)s", Dissertation Thesis for the Doctorate in Chemistry Sciences, University of Milan, 2007.

The synthesis of AGMA1 of formula (X) and ISA1 of formula (XI) are reported in J. Franchini et al., Biomacromolecules 2006, 7, 1215-1222 and S. Richardson et al., J. Drug Targeting 1999, 6, 391-404, respectively.

Applicants have found that contacting the surface of plates or other cell-supporting substrates with highly diluted solutions of soluble polyamidoamines according to the invention or, in general, with polymeric structures containing polyamidoamine segments, plates or cell-supporting substrates are obtained which allow excellent adhesion, proliferation and differentiation of cell lines and primary cell cultures, in particular glial and neuronal cells. The extent of cell adhesion, proliferation and differentiation obtained with such polyamidoamine treated plates or substrates is comparable or superior to that obtained using the poly-L-lysine or poly-L-ornithine commonly marketed for the same applications. The use of soluble polyamidoamines of the invention provides several advantages in comparison to the latter polymers, such as a reduced cost (form 1/30 to 1/200 of the cost of poly-L-lysine or poly-L-ornithine, depending on the particular polyamidoamine used) and straightforward operations resulting in a marked reduction (typically more than 50 percent) of the time necessary for substrate preparation using the currently used polyaminoacids. In addition, the soluble polyamidoamines of the invention (and derivatives thereof) are much more bio- and cyto-compatible than, for example, poly-L-lysine, with DL50 values from 1 to 3 order of magnitude higher. Finally, they are biodegradable to non toxic products.

In particular, using the soluble polyamidoamines according to the invention the procedures for the preparation of the plates or other cell-supporting substrates are simplified and made quicker than using standard basic polyamines for the following reasons:
1) plates or other cell-growth supporting substrates are treated with diluted solutions of soluble polyamidoamines, whose concentration may vary in a wider range than with the standard basic polyamines currently used;
2) removal of a possible excess of the soluble polyamidoamine can be obtained with a single washing or even without any washing step at all;
3) due to the biocompatibility and biodegradability of the soluble polyamidoamines the risks of toxic effects for cells, operators and environment are minimized.

Further advantages associated to the use of the soluble polyamidoamines according to the invention are the simple, easy to scale-up and environment friendly preparation of the products, which does not require organic solvents, heating or the use of catalysts. Finally, since polyamidoamines are a family of highly versatile polymers, with a great potential of structural variations, it is possible to prepare purposely tailored polymers able to support adhesion, growth and differentiation of cells of any histotype.

Examples of cell types that can conveniently be cultured using the soluble polyamidoamines of the invention include glial cells, namely microglia, cortical astrocytes, hippocampal astrocytes and primary neuronal hippocampal cells, PC12 cells, MDCK cells, adult stem cells (i.e. stem cells from human or rodent derived either from bone marrow or from adipose tissue), N2A murine neuroblastoma cells, U373MG human astrocytoma glioblastoma cells, T98-G cells and HEK293 human embryonic kidney cells.

Typically, concentrations from 0.1 to 5 mg/mL of soluble polyamidoamines are used. In the case of cultures of primary neuronal cells concentrations from 0.1 to 1 mg/mL are preferred. The concentration of 1 mg/mL is more preferably used for most of the cell lines.

Another object of this invention is to provide a process for the preparation of cell-growth supporting substrates functionalised with a soluble polyamidoamine, comprising the step of contacting an untreated cell-growth supporting substrate with a solution of a soluble polyamidoamine followed by an optional washing step, to provide a soluble polyamidoamine-functionalised substrate, wherein in the process the soluble polyamidoamine is used at a concentration of 0.1-5 mg/mL, preferably at the concentration of 1 mg/ml. In a preferred embodiment, no washing steps are required.

Still another object of this invention is to provide cell-growth supporting substrates, preferably cell-culture plate, functionalised with a soluble polyamidoamine.

Yet another object of this invention is to provide a kit containing a cell-growth supporting substrates, preferably a cell-culture plate, and a soluble polyamidoamine according to the invention.

The invention will now be described in more detail through the following examples.

### Example 1: preparation of substrates with mono-layers of soluble polyamidoamines

The substrate with mono layers of soluble polyamidoamines on glass coverslips was prepared as follows: coverslips were exposed overnight at 37°C to nitric acid, then abundantly washed in water (3 times, 2 hours per washing period). Coverslips were then dry sterilized at 180°C and placed in a sterile multiwell. Subsequently, coverslips were covered with previously solubilised polyamidoamine and after 20 minutes air dried for 2 hours under sterile conditions.

The deposition and the correct formation of mono-layers of polyamidoamines have been confirmed through Atomic Force Microscopy (AFM) and confocal microscopy. These techniques showed the uniform pattern of layer deposition, i.e. the lack of aggregates or of the exposure of the underlying plastic or glass support.

### Example 2: cell adhesion experiments of PC12 and MDCK cells

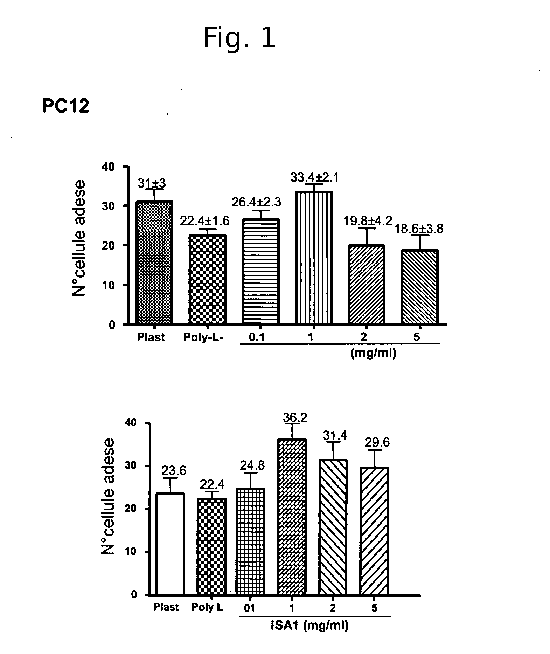

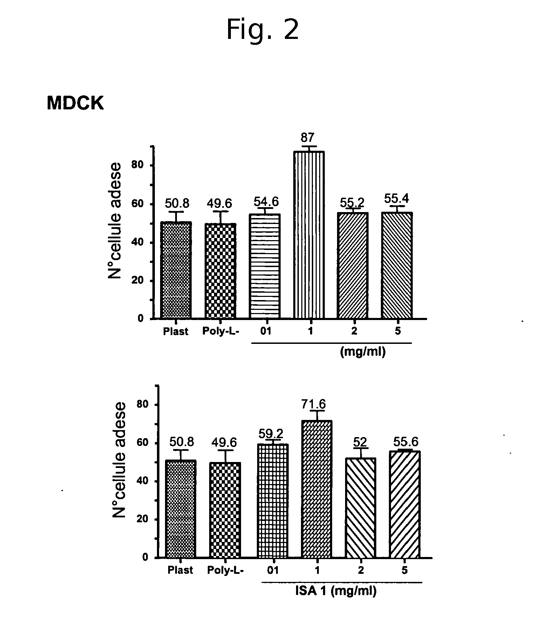

For the cell adhesion experiments, cells of the PC12 cell line (pheochromocytoma, *Rattus norvegicus*) or of the MDCK (Madin-Darby Canine Kidney) cell line were plated at a density of 90*10³ cell/cm² on glass or plastic substrates which were either unfunctionalised or had been functionalized with poly-L-lysine (1 mg/ml) or with the polyamidoamines AGMA1and ISA1 at different concentrations (0.1-1-2-5 mg/ml). The results obtained (Figure 1 and 2) show that using substrates functionalized with different types of polyamidoamines a 10-30% increase of adherent cells is observed after 2 and 24 hours from cell plating, in comparison to the use of poly-L-lysine functionalized substrates.

### Example 3: cell differentiation experiments of PC12 cells

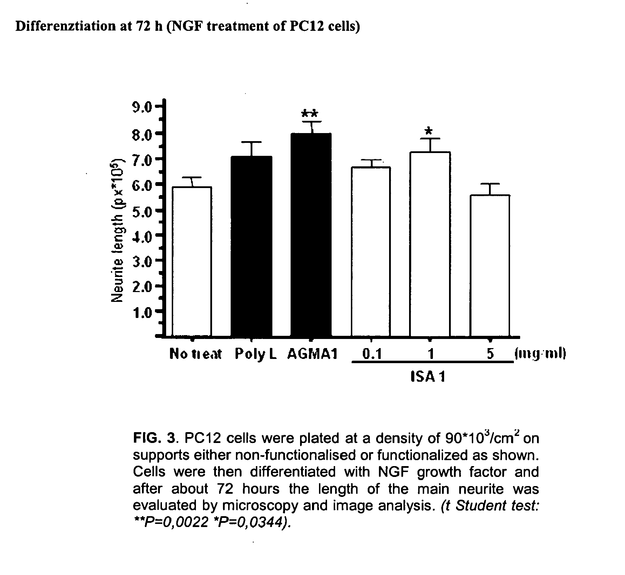

The evaluation of the cellular differentiation was performed using the PC 12 cells plated on glass or plastic substrates which were either unfunctionalised or had been functionalized with poly-L-lysine (1 mg/ml) or with the polyamidoamine ISA1 at different concentrations (0.1-1-2-5 mg/ml). After plating, cells were treated with the NGF growth factor for 72 h. As shown in Figure 3, the length of the neurites produced during cell differentiation on substrates functionalised with polyamidoamines was greater than that obtained using unfunctionalised substrates, whereas it was comparable to that obtained from neurites which underwent differentiation on poly-L-lysine functionalised substrates.

### Example 4: morphological analysis of PC12 cells

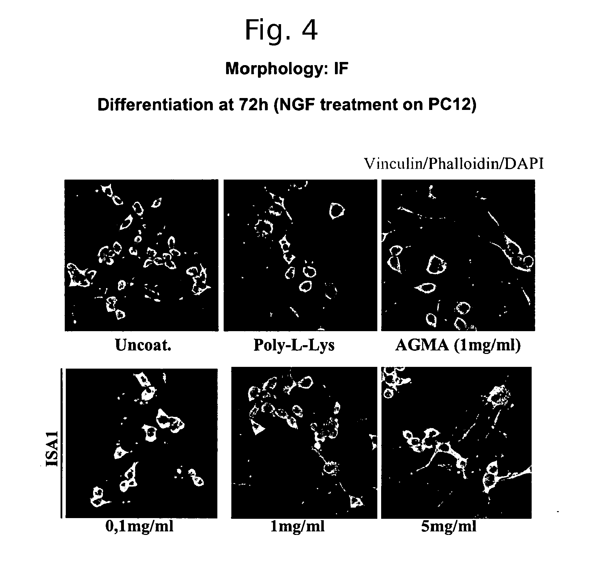

Morphological analyses were performed on NGF-differentiated PC12 cells using immunofluorescence techniques. The aim of this analysis was the evaluation of the levels of expression of the proteins Vinculine, which is involved in the cell adhesion processes and is a binding protein between the actin microfilaments and the cell membrane, and Phalloidin, which is a protein of the cytoskeleton. The results are reported in Figure 4. No modification was observed in the levels of expression of the proteins as assessed using anti-Vinculin and anti-Phalloidin antibodies. The results show that PC12 cells differentiate correctly on substrates functionalised with monolayers of the polyamidoamines AGMA1 or ISA1 in comparison to PC12 cells which underwent differentiation on poly-L-lysine functionalised substrates.

### Example 5: cell adhesion experiments of primary neuronal hippocampal cell cultures

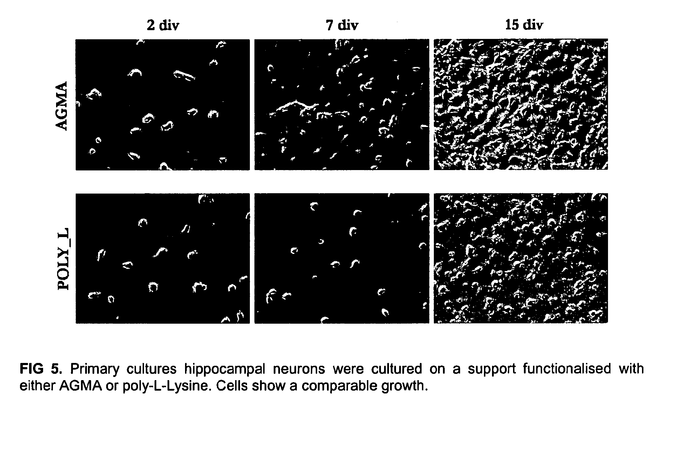

Cell adhesion experiments were performed on primary neuronal hippocampal cell cultures which had been plated in a substrate functionalized either with the polyamidoamine AGMA1 or with poly-L-Lysine. The neuronal growth was evaluated at different time points during cell culture and several parameters were considered, such as morphology, cell distribution on the surface of the substrate, adhesion density and vitality. The results are shown in Figure 5. No difference was observed in the growth of the cells plated in the substrate functionalized with agmatine or poly-L-lysine.

### Example 6: (Functional validation of primary neuronal hippocampal cell cultures)

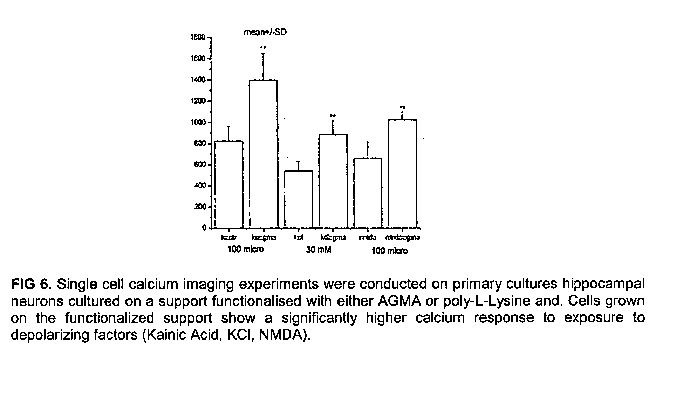

In order to evaluate the functional response of primary neurons grown either on the functionalized substrate as compared to their growth on poly-L-lysine, calcium currents following exposure to either kainic acid, KCl or NMDA were quantified by single cell calcium imaging experiments. Results indicated that at a few days in vitro, primary neuronal cultures grown on polyamidoamine-functionalized substrates appear to functionally respond with calcium increases in a significantly better way than cells cultured on poly-L-lysine substrates (Fig. 6).

## Claims

1. Use of soluble polyamidoamines having a molecular weight comprised from about 3.000 Da to about 120.000 Da, more preferably from about 5.000 Da to about 40.000 Da, to promote adhesion, growth and differentiation of cultured cell lines and primary cell cultures on cell-growth supporting substrates.

2. The use according to claim 1 wherein the soluble polyamidoamines are selected from basic polyamidoamines obtained by polyaddition of neutral bisacrylamides with amines and having from mild to strong cationic properties at pH 7.4.

3. The use according to claim 1 wherein the soluble polyamidoamines are selected from amphoteric polyamidoamines obtained by polyaddition of bisacrylamides substituted with acidic groups with amines, which have a prevailing acidic nature and an isoelectric point lower than 7.0.

4. The use according to claim 1 wherein the soluble polyamidoamines are selected from amphoteric polyamidoamines obtained by polyaddition of neutral or acidic-groups substituted bisacrylamides and aminoacids, which have a prevailing acidic nature and may have an isoelectric point lower than 7.0.

5. The use according to any of the preceding claims wherein the soluble polyamidoamines have as repeating units the structures of formulae (I)- (IX):

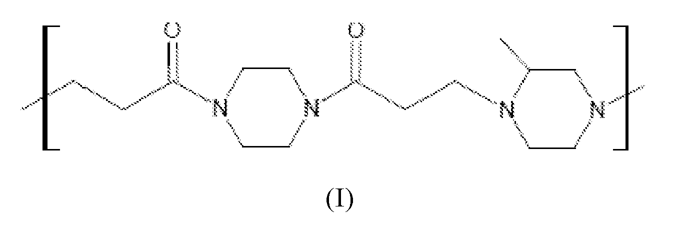

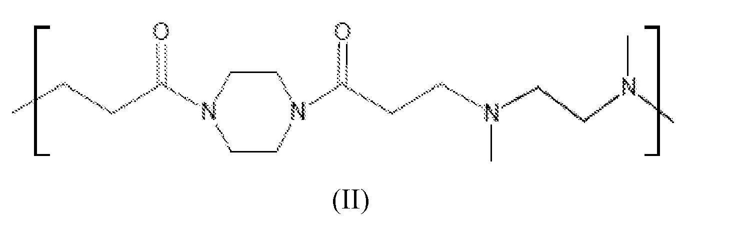

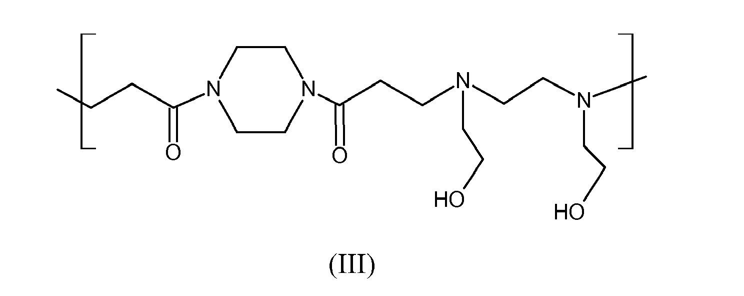

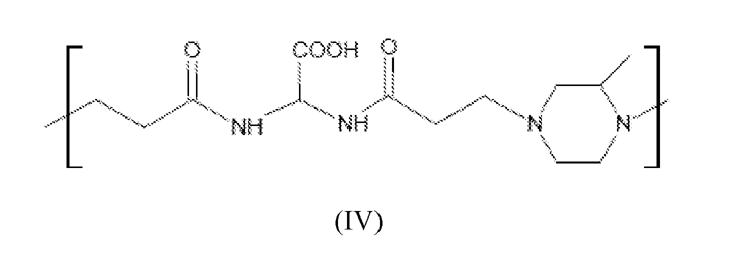

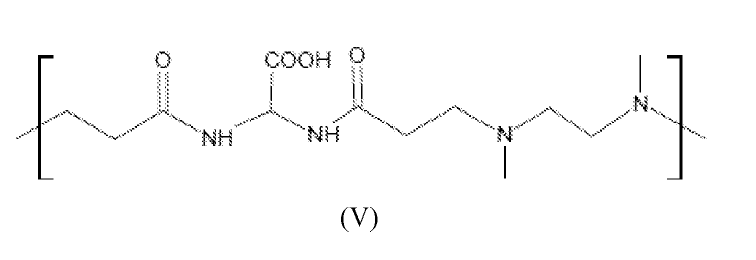

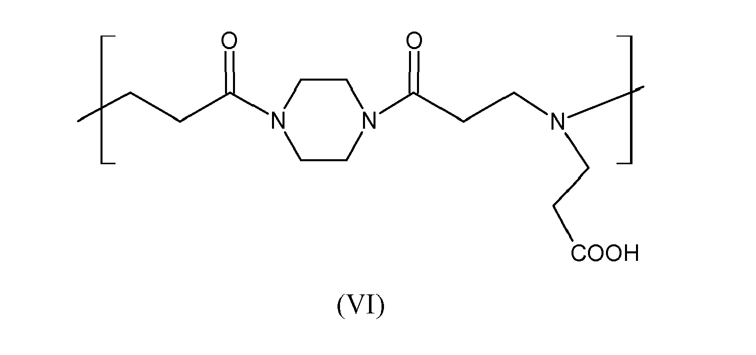

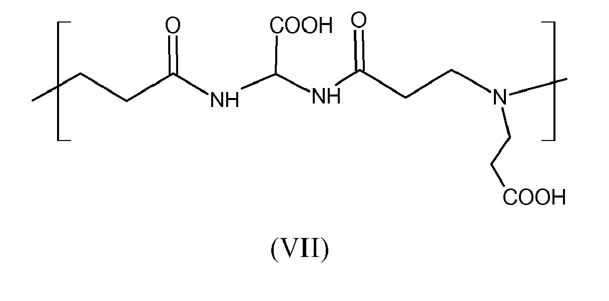

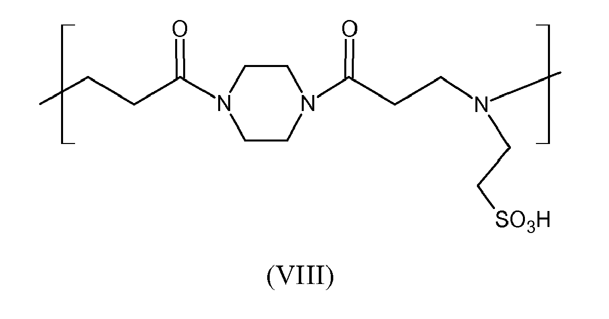

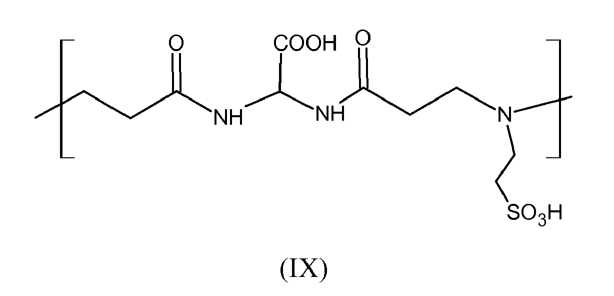

6. The use according to claim 1 wherein the soluble polyamidoamine is selected from polyamidoamines containing residues of agmatine, obtained by polyaddition of bisacrylamides substituted with neutral or acidic-groups and agmatine, which have a prevailing basic nature and, if amphoteric, may have an isoelectric point higher than 7.0.

7. The use according to claim 1 and 6 wherein the soluble polyamidoamine is AGMA1 of formula (X): 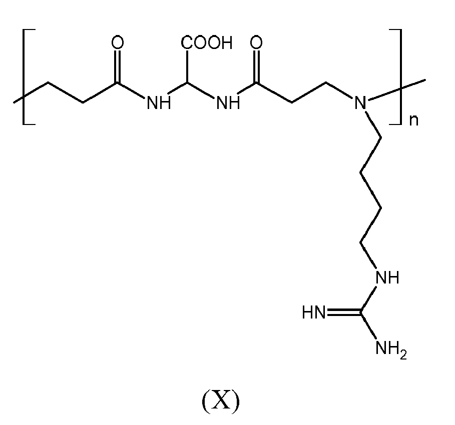 wherein n is an integer such that the molecular weight of AGMA1 is comprised from about 3.000 Da to about 120.000 Da, more preferably from about 5.000 Da to about 40.000 Da.

8. The use according to claim 1 wherein the soluble polyamidoamine is a copolymeric polyamidoamine called ISA1 of formula (XI): 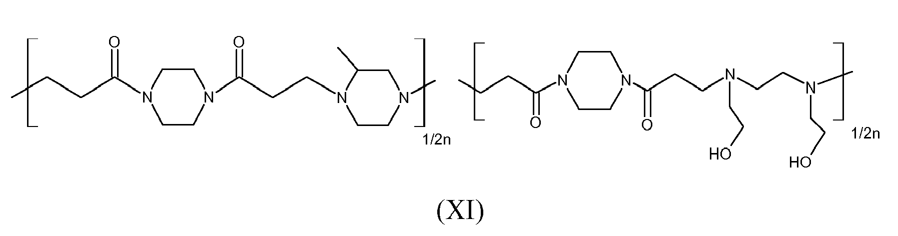 wherein n is an integer such that the molecular weight of the copolymeric ISA1 is comprised from about 3.000 Da to about 120.000 Da, more preferably from about 5.000 Da to about 40.000 Da.

9. The use according to claim 1 wherein the cultured cell lines are neuronal cells.

10. The use according to claim 1 wherein the cultured cells are selected from glial cells, in particular microglia, cortical astrocytes, hippocampal astrocytes and primary neuronal hippocampal cells, PC12 cells, MDCK cells, adult stem cells, N2A murine neuroblastoma cells, U373MG human astrocytoma glioblastoma cells, T798G cells and HEK293 human embryonic kidney cells.

11. A process for the preparation of cell-growth supporting substrates functionalised with a soluble polyamidoamine, comprising the step of contacting an untreated cell-growth supporting substrate with a solution of a soluble polyamidoamine, followed by an optional washing, to provide a soluble polyamidoamine-functionalised substrate.

12. The process according to claim 11 wherein the soluble polyamidoamine is used at a concentration of 0.1-5 mg/mL, preferably at the concentration of 1 mg/mL.

13. A process according to claims 11 or 12 wherein the cell-growth supporting substrate is a plate.

14. Cell-growth supporting substrates functionalised with a soluble polyamidoamine as defined in any one of claims from 1 to 8.

15. Kit containing a cell-growth supporting substrate, preferably a cell-culture plate, and a soluble polyamidoamine as defined in any one of claims from 1 to 8.
